# NEW EUROPEAN PATENT SPECIFICATION

(11) **EP 2 608 789 B2**
(45) Date of publication and mention of the opposition decision: **21.08.2024**
(45) Mention of the grant of the patent: 12.04.2017
(21) Application number: 11749331.2
(22) Date of filing: 22.08.2011
(51) Int. Cl.: A61K 31/495, A61P 25/00

(54) **THERAPEUTIC USES OF 1-[2-(2,4-DIMETHYL-PHENYLSULFANYL)PHENYL]PIPERAZINE**
THERAPEUTISCHE VERWENDUNG VON 1-[2-(2,4-DIMETHYL-PHENYLSULFANYL-)PHENYL-]PIPERAZIN
UTILISATIONS THÉRAPEUTIQUES DE 1-[2-(2,4-DIMÉTHYLPHÉNYLSULFANYL)PHÉNYL]PIPÉRAZINE

(30) Priority: 23.08.2010 US 375885 P; 23.08.2010 DK 201000739
(43) Date of publication of application: 03.07.2013
(73) Proprietor: H. Lundbeck A/S, 2500 Valby (DK)
(72) Inventor: DRAGHEIM, Marianne, DK-2950 Vedbæk (DK); FLOREA, Ioana, DK-2791 Dragør (DK)
(74) Representative: H. Lundbeck A/S
(86) International application number: PCT/DK2011/050317
(87) International publication number: WO 2012/025123

(56) References cited:
- WO-A1-2007/144005
- WO-A1-2009/062517
- US-A- 4 745 122
- KELLY KAREN ET AL: "Toward achieving optimal response: understanding and managing antidepressant side effects.", DIALOGUES IN CLINICAL NEUROSCIENCE 2008, vol. 10, no. 4, 2008, pages 409 - 418, XP009189114, ISSN: 1294-8322
- P. S. MASAND: "Weight gain associated with psychotropic drugs", E XP. OPIN. PHARMACOTHER, vol. 1, no. 3, 2000, pages 377 - 389, XP055452721
- ARTIGAS F ET AL: "P.2.c.040 A randomised, double-blind, placebo-controlled, active-referenced study of Lu AA21004 in patients with major depression", EUROPEAN NEUROPSYCHOPHARMACOLOGY, ELSEVIER SCIENCE PUBLISHERS BV, AMSTERDAM, NL, vol. 19, 1 September 2009 (2009-09-01), pages S426 - S427, XP026795343, ISSN: 0924-977X, [retrieved on 20090901]

## Description

### Field of the invention

The present invention relates to novel uses of 1-[2-(2,4-dimethyl-phenylsulfanyl)phenyl]-piperazine and pharmaceutically acceptable salts thereof in the treatment of CNS diseases.

### Background of the invention

1-[2-(2,4-dimethyl-phenylsulfanyl)phenyl]piperazine is a multimodal antidepressant that is thought to work through a combination of two pharmacological modes of action: reuptake inhibition and receptor activity. In vitro studies indicate that 1-[2-(2,4-dimethyl-phenylsulfanyl)-phenyl]piperazine is a 5-HT₃ and 5-HT₇ receptor antagonist, 5-HT_{1B} receptor partial agonist, 5-HT_{1A} receptor agonist and inhibitor of the 5-HT transporter. *In vivo* nonclinical studies have demonstrated that 1-[2-(2,4-dimethyl-phenylsulfanyl)phenyl]piperazine enhances levels of the neurotransmitters serotonin, noradrenalin, dopamine, acetylcholine and histamine in specific areas of the brain. All of these activities are considered to be of clinical relevance and potentially involved in the mechanism of action of the compound.

1-[2-(2,4-dimethyl-phenylsulfanyl)phenyl]piperazine and its use in the treatment of central nervous system (CNS) diseases were first disclosed in the International patent application published as WO 02/029232. Later patent applications (WO 2007/144005; WO 2008/113359; WO 2009/062517) have disclosed crystalline salts of the compound, manufacturing processes, and further therapeutic uses of the compound. Poster P.2.c.040 presented at the 22nd Congress of the European College of Neuropsychopharmacology, 12-16 September 2009, Istanbul, Turkey and poster NR4-024 presented at the 162nd Annual Meeting of the American Psychiatric Association, 16-21 May 2009, San Francisco, USA disclose the results from a proof-of-concept study to evaluate the efficacy and tolerability of the compound in patients with major depressive disorder (MDD). The results from this six weeks, randomised, placebo-controlled study with approximately 100 patients in each arm show that 1-[2-(2,4-dimethyl-phenylsulfanyl)phenyl]-piperazine separates significantly from placebo in the treatment of depressive and anxious symptoms in patients with MDD. The posters also report that no clinically relevant changes were seen in the clinical laboratory results, vital signs, weight, or ECG parameters. A full paper reporting the above mentioned proof-of-concept study authored by Alvares et al was made available on-line by Int. J. Neuropsychopharm. 18 July 2011.

Weight gain is a commonly observed adverse event for treatment with phycotropic drugs. The most pronounced effect seen is probably the weight gain associated with antipsychotics, such as clozapine and olanzapine, but clinically relevant weight gain is also observed for antidepressants [Exp. Opin. Pharmacother., 1, 377-389, 2000].

J.Clin.Psychpharm., 8, 323-330, 1988 reports that treatment with the tricyclic antidepressants (TCA) amitriptyline, nortriptyline, imipramine and desimiprmine gave rise to a dose-dependent weight gain of 0.57-1.37 kg/months. Similarly, J. Affective. Disord. 7, 133-138, 1984 reports that patients in treatment with the TCA s amitryptyline, nortryptyline or imipramine experienced a weight gain of 1.3-2.9 lbs/month (0.58- 1.31 kg/months). In fact, one half on the patients dropped out of the study due to excessive weight gain.

J.Clin.Psychiatry, 65, 1365-1371, 2004 reports on a 2.5 years study comparing weight gain in patients with obsessive-compulsive disorder (OCD) being treated with various antidepressants. In this study (as in many others), an increase in weight is considered potentially clinically significant (PCS) if it is 7% or above compared to baseline level. At the end of the study period the following percentages of the patients had a PCS weight increase: clomiptramine (34.8%), citalopram (14.3%), fluoxetine (8.7%), fluvoxamine (10.7%), paroxetine (14.3%) and sertraline (4.5%).

Int. Clin. Psychpharm., 13, 63-73, 1998 reports on a relapse prevention study comparing the long-term (up to two years) effects of the antidepressants mirtazapine and amitryptyline. The proportion of patients showing a PCS increase in body weight was 22% with amitriptyline, 12.7% with mirtazapine and 3.6% with placebo.

J.Clin.Psychiatry, 61, 863-867, 2000 reports on a long-term study (26-32 weeks) comparing the weight effects of the antidepressants fluoxetine, sertraline and paroxetine. The proportion of patients showing a PCS increase in body weight was 25.5% with paroxetine, 4.2% with sertraline and 6.6% with fluoxetine. These observations are supported by anecdotal reports [J.Clin.Psychiatry 60 (suppl 21), 16-19, 1999] that 25-33% of patients on maintenance treatment (i.e. long-term) with serotonin re-uptake inhibitors (SSRI) gain a substantial amount of weight and that paroxetine may be more likely than other drugs in this class to induce weight gain.

On the other hand, a number of short-term studies with paroxetine report an absence of weight gain or even weight loss. Acta Psychiatr. Scand. 80 (suppl 350), 117-123, 1989 reports that no weight gain was noted on a six week study with paroxetine. Am. J. Psychiatry, 160, 749-756, 2003 reports a small weight loss in a 566 patients, eight weeks study with paroxetine. J Clin. Psychiatry, 62, 350-536, 2001 discloses that no substantial effects on weight were observed in a 324 patients, eight weeks trial with paroxetine.

Am.J. Psychiatry, 156, 1170-1176, 1999 reports on a long-term (one year) study on the body weight effects of fluoxetine. While fluoxetine in the acute phase induce a slight weight decrease, the long-term effect on weight is similar to placebo.

Finally, Clin. Therapeutics, 24, 662-672, 2002 reports on the long-term (52 weeks) body weight effects of bupropion. Bupripion gives rise to a modest, but significant decrease in body weight.

The above literature references show that it is difficult to predict the effects on weight associated with treatment with antidepressants. Many antidepressants do have weight gain as an adverse event, others don t. Also, it appears that it is difficult to predict the weight effects associated with long-term treatment with an antidepressant from results reported from short-term studies. This unpredictability is likely to be explained by at least two factors. Firstly, notwithstanding the severity of weight gain related to treatment with antidepressants, it is normally of a smaller magnitude than for treatment with antipsychotics. Thus, any changes in weight may take longer time to develop and become evident. Secondly, weight loss or gain may in itself be a symptom of depression. Therefore, treatment emergent weight gain (i.e. weight gain associated with the compound as such) may be confounded with weight changes resulting from the treatment of the disease; hence it may take longer time to separate the two effects. These observations seem to be supported by a recently published meta-analysis of 116 clinical studies on the effect of antidepressants on body weight [J.Clin.Psych.71, 1259-1272, 2010].

US 40475,122 discloses the use of paroxetine for the treatment of obesity.

Dialog Clin Neurosci 10, 409-418, 2008 discloses that weight related adverse events associated with treatment with serotonin and noradrenaline reuptake inhibitors are not present at the beginning of the treatment but emerge over time.

Given the important implications treatment emergent weight gain may have on patients, it is important to be able to provide treatments with antidepressants associated with no or only few weight related adverse events.

### Summary of the invention

The present inventors have surprisingly found that long-term treatment with 1-[2-(2,4-dimethyl-phenylsulfanyl)phenyl]piperazine and pharmaceutically acceptable salts thereof (Compound I) is not associated with weight increase. Thus, in one embodiment, the invention relates to Compound I for use in the long-term treatment of depression or anxiety in a patient who has previously received medication for the treatment of said disease which medication was ceased due to weight gain related adverse events, wherein long-term treatment refers to a treatment period above 12 weeks.

In one embodiment, the invention provides Compound I for use in the manufacture of a medicament for the long-term treatment of depression or anxiety in a patient who has previously received medication for the treatment of said disease which medication was ceased due to weight gain related adverse events, wherein long-term treatment refers to a treatment period above 12 weeks.

### Detailed description of the invention

The molecular structure of 1-[2-(2,4-dimethyl-phenylsulfanyl)phenyl]piperazine is shown below

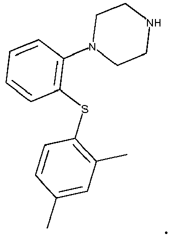

In the present context, 1-[2-(2,4-dimethyl-phenylsulfanyl)phenyl]piperazine and pharmaceutically acceptable salts thereof is referred to as Compound I. A particular salt, e.g. the hydrobromide salt is referred to as Compound I HBr.

In one embodiment, said pharmaceutically acceptable salts are acid addition salts of acids that are non-toxic. Said salts include salts made from organic acids, such as maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, mandelic, cinnamic, citraconic, aspartic, stearic, palmitic, itaconic, glycolic, p-aminobenzoic, glutamic, benzenesulfonic, theophylline acetic acids, as well as the 8-halotheophyllines, for example 8-bromotheophylline. Said salts may also be made from inorganic salts, such as hydrochloric, hydrobromic, sulfuric, sulfamic, phosphoric and nitric acids. Particular mention is made of salts made from methanesulfonic acid, maleic acid, fumaric acid, meso-tartaric acid, (+)-tartaric acid, (-)-tartaric acid, hydrochloric acid, hydrobromic acid, sulphuric acid, phosphorous acid and nitric acid. Distinct mention is made of the hydrobromide salt.

Oral dosage forms, and in particular tablets, are often preferred by the patients and the medical practitioner due to the ease of administration and the consequent better compliance. For tablets, it is preferable that the active ingredients are crystalline. In one embodiment, the invention relates to the use of Compound I in a crystalline form. The crystallinity of Compound I is evidenced by the XRDP shown in e.g WO 2007/144005.

As evidenced by the data provided in WO 2007/144005 (see example 4a-f) the hydrobromide salt of 1-[2-(2,4-dimethylphenylsulphanyl)-phenyl]piperazine (Compound I HBr) may exist in several forms, i.e. it is polymorphic. The polymorphic forms have different properties. The beta form Compound I HBr (as defined in WO 2007/144005) is the more stable as demonstrated by the higher DSC melting point and the lower solubility. Moreover, the beta form has an attractive combination of low hygroscopicity and solubility, which makes this compound particularly suited for making tablets. Hence, in one embodiment, the invention provides the use of the hydrobromide salt of 1-[2-(2,4-dimethylphenylsulphanyl)-phenyl]piperazine with XRDP reflections at approximately 6.89, 9.73, 13,78 and 14.62 (°2θ) (± 0.1), such as at approximately 6.89, 8.48, 9.73, 13.78, 14.62 and 24.73 (°2θ) (± 0.1).

Alternatively, if Compound I is provided in an oral drop formulation, other salts characterised by higher solubility may be preferred. When a compound is administered as an oral drop formulation, a few drops of a concentrated, liquid formulation of said drug is measured out and added to a glass of water, juice or the like that the patient drinks. As an example, the antidepressant cipramil is provided as an oral drop formulation at 40 mg/ml. The DL lactate salt of Compound I has been found to have a high solubility and therefore to be particularly suited for oral drop formulations. Hence, in one embodiment, the invention provides the use of the DL-lactate salt of 1-[2-(2,4-dimethylphenylsulphanyl)-phenyl]piperazine.

There are numerous reasons why weight gain should be avoided in general and in relation with the treatment of CNS diseases in particular. Obesity and overweight is associated with serious conditions, such as type II diabetes mellitus, hypertension and coronary heart diseases. A person who gains 5-7.9 kg as adult is 1.9 times more likely to develop type II diabetes mellitus than a person who maintains a stable weight after the age of 18 [J. Clin. Psych., 1999, 60(suppl 21), 5-9, 1999]. Hence, a primary focus in the treatment of diseases, such as type II diabetes mellitus, hypertension and coronary heart diseases is a dietary effort in order to make the patient lose weight. In more general terms, weight gain is associated with impaired physical functioning, reduced quality of life and poor mental health. Treatment compliance is a further consideration. Due to the physical discomfort, the reduced self esteem and the above discussed increased health risk associated with weight increase many patients who experience treatment emergent weight gain are reluctant to take the drug as prescribed with the consequent poor treatment outcome. Hence, weight gain associated with treatment of CNS diseases is an essential factor and it is important to be able to offer a treatment of CNS diseases with no or only a few weight associated adverse events.

As shown in the examples, Compound I is not associated with weight gain in long-term treatment. Therefore, Compound I is particularly well suited for long-term treatment, treatment of patients who cannot accept other medication due to weight related adverse events (i.e. as 2^{nd} line treatment), for patients who in addition to their CNS disease suffer from a disease wherein it is important to avoid weight gain, and in patients that are overweight.

In the present context, long-term is intended to include maintenance treatment, and it is characterised by a treatment period above 12 weeks, such as above 24 weeks, such as above 48 weeks, such as above 72 weeks. Long-term treatment includes chronic treatment, such as lifelong treatment.

In one embodiment, the invention provides Compound I for use in the long-term treatment of depression or anxiety in a patient who has previously received medication for the treatment of said disease which medication was ceased due to weight gain related adverse events, wherein long-term treatment refers to a treatment period above 12 weeks. Weight gain associated adverse events include hypertension, increased blood sugar level and decreased self esteem or quality of life. In particular, said adverse event is weight gain. The previously received medication is typically a psychotropic, such as an antipsychotic or an antidepressant.

According to its unique pharmacological profile, Compound I is expected to be useful in the treatment of a various CNS diseases. The International patent applications published as WO 03/029232, WO 2007/144005, WO 2008/113359 and WO 2009/062517 disclose examples of such CNS diseases. In particular, CNS disease as used in the present context denotes a disease selected from depression disorders, anxiety disorders, chronic pain and abuse.

Depression disorders include major depressive disorder, dysthymic disorder, depression associated with bipolar disorder and depression with anxious features (anxious depression). Anxiety disorders include generalised anxiety disorder, panic attacks, phobias, obsessive-compulsive disorder and post-traumatic stress disorder. Chronic pain includes phantom limb pain, neuropathic pain, diabetic neuropathy, post-herpetic neuralgia, carpal tunnel syndrome, HIV neuropathy, migraine, tension-type headache, and fibromyalgia syndrome. Abuse includes craving for or abuse of alcohol, narcotics and drugs.

In one embodiment, the invention provides the use of Compound I in the manufacture of a medicament for the long-term treatment of depression or anxiety in a patient who has previously received medication for the treatment of said disease which medication was ceased due to weight gain related adverse events, wherein long-term treatment refers to a treatment period above 12 weeks. Weight gain associated adverse events include hypertension, increased blood sugar level and decreased self esteem or quality of life. In particular, said adverse event is weight gain. The previously received medication is typically a psychotropic, such as an antipsychotic or an antidepressant.

A "therapeutically effective amount" of a compound as used herein means an amount sufficient to cure, alleviate or partially arrest the clinical manifestations of a given disease and its complications in a therapeutic intervention comprising the administration of said compound. An amount adequate to accomplish this is defined as "a therapeutically effective amount". Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatment are two separate aspects of the invention. The patient to be treated is preferably a mammal, in particular a human being.

Compound I is typically administered in daily doses of 1-100 mg, such as 2- 40 mg, such as 2, 5, 10, 15, 20, 25, 30, 35 mg. A daily dose may involve once daily dosing, or dosing two or more times daily.

Compound I is conveniently administered as a pharmaceutical composition which may be prepared by conventional methods in the art. Particular mentioning is made of tablets, which may be prepared by mixing the active ingredient with ordinary adjuvants and/or diluents and subsequently compressing the mixture in a conventional tabletting machine. Examples of adjuvants or diluents comprise: anhydrous calcium hydrogen phosphate, PVP, PVP-VA copolymers, microcrystalline cellulose, sodium starch glycolate, corn starch, mannitol, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.

Solutions for injections may be prepared by dissolving the active ingredient and possible additives in a part of the solvent for injection, preferably sterile water, adjusting the solution to desired volume, sterilising the solution and filling it in suitable ampules or vials. Any suitable additive conventionally used in the art may be added, such as tonicity agents, preservatives, antioxidants, etc.

Oral drop formulations typically comprise, in addition to the active ingredient, excipients selected from solvent, buffer, surfactant, surface tension modifier, viscosity modifier, preservative, antioxidant, colourants, tastes masker, and flavour.

The pharmaceutical compositions which may be used in this invention may be administered by any suitable route, for example orally in the form of tablets, capsules, powders, syrups, liquids etc., or parenterally in the form of solutions for injection. For preparing such compositions, methods well known in the art may be used, and any pharmaceutically acceptable carriers, diluents, excipients or other additives normally used in the art may be used.

Conveniently, Compound I is administered in unit dosage form containing said compound in an amount of about 1 to 50 mg, such as 5, 10, 15 or 20 mg. Tablets comprising Compound I may conveniently be prepared by wet granulation. Using this method, the dry solids (active ingredients, filler, binder etc.) are blended and moistened with water or another wetting agent (e.g. an alcohol) and agglomerates or granules are built up of the moistened solids. Wet massing is continued until a desired homogenous particle size has been achieved whereupon the granulated product is dried. Compound I is typically mixed with lactose monohydrate, corn starch and copovidone in a high shear mixer together with water. Following formation of granulates, these granulates may be sieved in a sieve with a suitable sieve size, and dried. The resulting, dried granulates are then mixed with microcrystalline cellulose, croscarmellose sodium and magnesium stearate, following which the tablets are pressed. Alternatively, wet granulation of Compound I may be achieved using mannitol, corn starch and copovidone, which granulates are mixed with microcrystalline cellulose, sodium starch glycolate and magnesium stearate before tablets are pressed. Alternatively, wet granulation of Compound I may be achieved by using anhydrous calcium hydrogen phosphate, corn starch and copovidone, which granulates are mixed with microcrystalline cellulose, sodium starch glycolate (type A), talc and magnesium stearate before tablets are pressed. Copovidone is a PVP-VA copolymer. Alternatively, tablets comprising Compound I may be obtained by mixing Compound I, mannitol and microcrystalline cellulose in a fluid bed granulation dryer onto which mixture an aqueous solution of hydroxypropyl cellulose is sprayed to give a granulated powder. The obtained granulates are then mixed with microcrystalline cellulose, sodium starch glycolate and magnesium stearate. The obtained mixture can subsequently be pressed into tablets. Typically, the tablets are coated with a suitable coating material.

The use of the terms a and an and the and similar referents in the context of describing the invention are to be construed to cover both the singular and the plural, unless otherwise indicated herein or clearly contradicted by context. For example, the phrase "the compound" is to be understood as referring to various compounds used in the invention or particular described aspect, unless otherwise indicated.

Unless otherwise indicated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or aspect of the invention using terms such as comprising , having, including, or containing with reference to an element or elements is intended to provide support for a similar aspect or aspect of the invention that consists of , consists essentially of , or substantially comprises that particular element or elements, unless otherwise stated or clearly contradicted by context (e.g., a composition described herein as comprising a particular element should be understood as also describing a composition consisting of that element, unless otherwise stated or clearly contradicted by context).

### Examples

### Example 1 Long-term effects on body weight in MDD patients

The effects on weight associated with treatment with 1-[2-(2,4-dimethyl-phenylsulfanyl)phenyl]piperazine HBr was investigated in a relapse prevention study in patients with major depressive disorder. Approximately 600 patients were enrolled in an open-label study in which they received 5 or10 mg Compound I HBr (calculated as free base). After 12 weeks, responders (N∼400) were randomised to placebo or 5/10 mg Compound I HBr for a 24 weeks double-blind placebo-controlled study. The recruitment of patients took place over time, and since the end of the study was defined by the last patient having completed the 24 weeks study, some patients received up to 64 weeks double-blind placebo-controlled treatment. This study is also reported in poster NR4-14 presented at the 164th Annual Meeting of the American Psychiatric Association, May 14-18 2011, Honolulu, Hawaii, USA.

The tables below show the mean difference in weight relative to the start of the open label period and to the time of randomisation and the fraction of patients experiencing equal to or more than 7% weight gain during the study, i.e. potentially clinically significant increase.

**Tabel 1 Difference in weight relative to start of open label period (t=0)**

| Treatment | Week | Number of patients | Δ weight (kg) | ≥7% weight gain (%) |
|---|---|---|---|---|
| Placebo | 12 | 192 | 0.4 | 0.5 |
| Placebo | 24 | 188 | 0.9 | 4.8 |
| Placebo | 36 | 136 | 0.8 | 3.7 |
| Placebo | 48 | 66 | 0.3 | 4.5 |
| Placebo | 60 | 28 | 0.9 | 10.7 |
| Placebo | 72 | 12 | 0.0 | 8.3 |
| Placebo | 76 | 3 | 4.3 | 33.3 |
| | | | | |
| Compound | 12 | 204 | 0.0 | 2.0 |
| Compound | 24 | 199 | 0.3 | 6.5 |
| Compound | 36 | 157 | 0.6 | 8.9 |
| Compound | 48 | 90 | 0.7 | 8.9 |
| Compound | 60 | 52 | 0.4 | 9.6 |
| Compound | 72 | 19 | -0.2 | 10.5 |
| Compound | 76 | 3 | -2.3 | 0.0 |

**Tabel 2 Weight difference relative to time of randomisation (t=12 weeks)**

| Treatment | Week | Number of patients | Δ weight (kg) | ≥7% weight gain (%) |
|---|---|---|---|---|
| Placebo | 24 | 188 | 0.5 | 2.7 |
| Placebo | 36 | 136 | 0.3 | 3.7 |
| Placebo | 48 | 66 | 0.1 | 4.5 |
| Placebo | 60 | 28 | 1.1 | 7.1 |
| Placebo | 72 | 12 | 0.2 | 16.7 |
| Placebo | 76 | 3 | 4.9 | 66.7 |
| | | | | |
| Compound | 24 | 199 | 0.4 | 2.0 |
| Compound | 36 | 157 | 0.6 | 6.4 |
| Compound | 48 | 90 | 0.9 | 8.9 |
| Compound | 60 | 52 | 0.8 | 13.5 |
| Compound | 72 | 19 | 0.3 | 10.5 |
| Compound | 76 | 3 | -0.8 | 0.0 |

When the data in table 1 are evaluated it should be kept in mind that patients in the placebo arm received active treatment for the first 12 weeks. Nevertheless, the data presented in tables 1 and 2 clearly show that even after long-term treatment there is no meaningful difference between placebo and 1-[2-(2,4-dimethyl-phenylsulfanyl)phenyl]piperazine HBr as far as weight gain in concerned.

### Example 2 Long-term effects on body weight in GAD patients

The effects on weight associated with treatment with 1-[2-(2,4-dimethyl-phenylsulfanyl)phenyl]piperazine HBr was investigated in a relapse prevention study in patients with generalised anxiety disorder. 687 patients were enrolled in an open-label study in which they received 5 or10 mg Compound I HBr (calculated as free base). After 20 weeks, responders to treatment (N=459) were randomised to placebo or 5/10 mg Compound I HBr for a 24-56 weeks double-blind placebo-controlled study. The recruitment of patients took place over time, and since the end of the study was defined by the last patient having completed at least 24 weeks, some patients received up to 56 weeks double-blind placebo-controlled treatment.

The tables below show the mean difference in weight relative to the start of the open label period and to the time of randomisation and the fraction of patients experiencing equal to or more than 7% weight gain during the study, i.e. potentially clinically significant increase.

**Tabel 3 Difference in weight relative to start of open label period (t=0)**

| Treatment | Week | Number of patients | Δ weight (kg) | ≥7% weight gain (%) |
|---|---|---|---|---|
| Placebo | 20 | 227 | 0.1 | 4.0 |
| Placebo | 32 | 222 | 0.3 | 5.9 |
| Placebo | 44 | 153 | 0.4 | 8.5 |
| Placebo | 56 | 72 | 0.3 | 9.7 |
| Placebo | 68 | 20 | 0.3 | 5.0 |
| Placebo | 76 | 3 | 2.0 | 0.0 |
| | | | | |
| Compound | 20 | 227 | 0.3 | 2.6 |
| Compound | 32 | 220 | 0.5 | 3.6 |
| Compound | 44 | 179 | 0.8 | 8.4 |
| Compound | 56 | 84 | 1.5 | 17.9 |
| Compound | 68 | 28 | 1.5 | 10.7 |
| Compound | 76 | 4 | 0.9 | 0.0 |

**Tabel 4 Weight difference relative to time of randomisation (t=20 weeks)**

| Treatment | Week | Number of patients | Δ weight (kg) | ≥7% weight gain (%) |
|---|---|---|---|---|
| Placebo | 32 | 223 | 0.2 | 2.7 |
| Placebo | 44 | 154 | 0.4 | 5.8 |
| Placebo | 56 | 72 | 0.3 | 5.6 |
| Placebo | 68 | 20 | 0.5 | 0.0 |
| Placebo | 76 | 3 | 1.6 | 0.0 |
| | | | | |
| Compound | 32 | 221 | 0.3 | 0.9 |
| Compound | 44 | 180 | 0.5 | 4.4 |
| Compound | 56 | 84 | 1.1 | 10.7 |
| Compound | 68 | 28 | 1.7 | 10.7 |
| Compound | 76 | 4 | 1.6 | 0.0 |

When the data in table 3 are evaluated it should be kept in mind that patients in the placebo arm received active treatment for the first 20 weeks. The combined data presented in tables 1-4 show that even after long-term treatment there is no meaningful difference between placebo and 1-[2-(2,4-dimethyl-phenylsulfanyl)phenyl]piperazine HBr as far as weight gain in concerned.

## Claims

1. 1-[2-(2,4-dimethyl-phenylsulfanyl)phenyl]piperazine and pharmaceutically acceptable salts thereof for use in the long-term treatment of depression or anxiety in a patient who has previously received medication for the treatment of said disease which medication was ceased due to weight gain related adverse events, wherein long-term treatment refers to a treatment period above 12 weeks.

2. 1-[2-(2,4-dimethyl-phenylsulfanyl)phenyl]piperazine and pharmaceutically acceptable salts thereof according of claim 1 which is the hydrobromide salt.

3. Use of 1-[2-(2,4-dimethyl-phenylsulfanyl)phenyl]piperazine and pharmaceutically acceptable salts thereof in the manufacture of a medicament for the long-term treatment of depression or anxiety in a patient who has previously received medication for the treatment of said disease which medication was ceased due to weight gain related adverse events, wherein long-term treatment refers to a treatment period above 12 weeks.

4. The use according to claim 3, wherein said pharmaceutically acceptable salt is the hydrobromide salt.

## Patentansprüche

1. 1-[2-(2,4-Dimethyl-phenylsulfanyl)phenyl]piperazin und pharmazeutisch verträgliche Salze davon zur Verwendung in der langfristigen Behandlung von Depression oder Angst bei einem Patienten, der zuvor Medikamente für die Behandlung der Krankheit erhalten hat, wobei die Medikamente aufgrund von mit Gewichtszunahme zusammenhängenden unerwünschten Nebenwirkungen eingestellt wurden, wobei sich die langfristige Behandlung auf eine Behandlungsdauer von mehr als 12 Wochen bezieht.

2. 1-[2-(2,4-Dimethyl-phenylsulfanyl)phenyl]piperazin und pharmazeutisch verträgliche Salze davon gemäß Anspruch 1, welches das Hydrobromidsalz ist.

3. Verwendung von 1-[2-(2,4-Dimethyl-phenylsulfanyl)phenyl]piperazin und pharmazeutisch verträgliche Salze davon in der Herstellung eines Medikaments für die langfristige Behandlung von Depression oder Angst bei einem Patienten, der zuvor Medikamente für die Behandlung der Krankheit erhalten hat, wobei die Medikamente aufgrund von mit Gewichtszunahme zusammenhängenden unerwünschten Nebenwirkungen eingestellt wurden, wobei sich die langfristige Behandlung auf eine Behandlungsdauer von mehr als 12 Wochen bezieht.

4. Verwendung gemäß Anspruch 4, wobei das pharmazeutisch verträgliche Salz das Hydrobromidsalz ist.

## Revendications

1. 1-[2-(2,4-diméthyl-phénylsulfanyl)phényl]pipérazine et ses sels pharmaceutiquement acceptables pour utilisation dans le traitement à long terme de la dépression ou de l'anxiété chez un patient qui a précédemment reçu une médication pour le traitement de ladite maladie, laquelle médication a été interrompue en raison d'événements indésirables dus au poids, dans laquelle le traitement à long terme se réfère à une période de traitement supérieure à 12 semaines.

2. 1-[2-(2,4-diméthyl-phénylsulfanyl)phényl]pipérazine et ses sels pharmaceutiquement acceptables selon la revendication 1 qui est le sel de bromhydrate.

3. Utilisation de 1-[2-(2,4-diméthyl-phénylsulfanyl)phényl]pipérazine et de ses sels pharmaceutiquement acceptables dans la fabrication d'un médicament pour le traitement à long terme de la dépression ou de l'anxiété chez un patient qui a précédemment reçu une médication pour le traitement de ladite maladie, laquelle médication a été interrompue en raison d'événements indésirables dus au poids, dans laquelle le traitement à long terme se réfère à une période de traitement supérieure à 12 semaines.

4. Utilisation selon la revendication 3, dans laquelle ledit sel pharmaceutiquement acceptable est le sel de bromhydrate.
